# EUROPEAN PATENT APPLICATION

(11) **EP 4 557 308 A1**
(43) Date of publication of application: **21.05.2025**
(21) Application number: 23383175.9
(22) Date of filing: 17.11.2023
(51) Int. Cl.: G16H 50/20, G16H 50/30, G16H 50/70

(54) **EVALUATION SYSTEM FOR ESTABLISHING RISK PHENOTYPES ON PREHOSPITAL CARE**

(71) Applicant: Universidad de Valladolid, 47002 Valladolid (ES); Gerencia Regional de Salud de Castilla y León, 47007 Valladolid (ES); Universidad de Castilla La Mancha, 02071 Albacete (ES)
(72) Inventor: MARTIN RODRÍGUEZ, Francisco, 47005 Valladolid (ES); LÓPEZ IZQUIERDO, Raul, 47012 Valladolid (ES); DEL POZO VEGAS, Carlos, 47003 Valladolid (ES); SANZ GARCÍA, Ancor, 45600 Toledo (ES)
(74) Representative: Hoffmann Eitle

(57) **Abstract**

The present invention refers to an evaluation system for establishing risk phenotypes on a prehospital care.

## Description

### FIELD OF THE INVENTION

The present invention refers to the medical field. Particularly, it refers to an evaluation system for establishing risk phenotypes on prehospital care.

### STATE OF THE ART

Emergency Medical Services (EMS) must manage acute life-threatening illness as part of the standard workflow. EMS-providers in dynamic and critical scenarios must perform timely decision-making without delay. In fact, the quick targeting of high-risk patients represents a major challenge in prehospital care.

EMS has continuously implemented different strategies to improve the timely recognition of high-risk patients to provide appropriate prehospital critical care. In this sense, the application of scores, biomarkers, risk-models, etc., is becoming a routine clinical practice.

Point-of-care testing (POCT) is capable to perform blood tests, including, among others, venous or arterial blood gases, renal profile, glucose, lactate, hematocrit, hemoglobin, troponin, dimer D, myoglobin, and international normalized ratio. In few minutes, the healthcare personnel can dispose of bedside analytical data, formerly reserved for hospital use exclusively, helping and supporting the on-scene decision-making process. In the case of patients without a clear acute life-threatening illness, on-scene blood tests may assist in screening for hidden high-risk conditions, e.g., electrolyte disturbances, metabolic-endocrine diseases, respiratory failure, anemia, or renal insufficiency.

In addition, precision emergency medicine is acquiring importance day-by-day. In prehospital critical care, early warning scores, risk scales, and predictive models, to detect time-dependent diseases and their short- and long-term prognosis are commonplace. Likewise, phenotypes are more and more commonly used to identify certain pathophysiological conditions in hospital scope, starting to extend to the prehospital setting.

There is an unmet medical need of identifying phenotypes in prehospital care. Consequently, the purpose of the present invention is to develop clustering-derived phenotypes in patients with acute life-threatening illness, based on variables collected by EMS (vital signs and biomarkers) at zero minute of initial emergency care. And to evaluate the short- and medium-term prognosis, and the diseases associated to each phenotype.

### DESCRIPTION OF THE INVENTION

### Brief description of the invention

Since EMS everyday face critical situations that require patients risk classification based on analytical and vital signs, the present invention is focused on stablishing clustering-derived phenotypes based on prehospital analytical and vital signs that allow a risk stratification.

The present invention paves the way for applying standardized prehospital laboratory tests and routine vital signs to derive bedside phenotypes. Phenotyping to target critical care and support the decision-making process, has become a regular element of clinical practice. Nevertheless, in prehospital critical care phenotyping is emerging tentatively.

Based on 30 variables (epidemiological, clinical, and analytical biomarkers) collected on a prehospital care, blind to the main dependent outcome, three clustering-derived phenotypes have been identified in the present invention. *Alpha* phenotype was characterized by a compromised clinical condition (tachypnea, desaturation, impaired SaFi, lower blood pressure, tachycardia, and a poorer consciousness level), associated with acidosis, hypercapnia, negative base excess, hyperlactacidemia, abnormal renal profile (creatinine and blood urea nitrogen rises) and hyperglycemia. Patients from *Alpha* phenotype presented a marked dependence over time for on-scene life support interventions, the largest rates of ICU-admissions, and mortality (3-times higher as compared to *Beta* phenotype and 10-times more vs *Gamma* phenotype). *Beta* phenotype reported improved acid-base balance, increased blood oxygen, mild-hyperlactacidemia, renal profile returned to target ranges, and mild-hyperglycemia. *Gamma* phenotype, in contrast, reported results within ranges regarding all variables. The most accentuated difference between Beta vs Gamma phenotype was 30-day mortality rate (10.2% vs 3.2%, respectively).

Regarding the suspected prehospital diagnoses, *Alpha* phenotype was characterized by cardiac arrest, heart failure (including congestive heart failure) and dyspnea, followed by febrile syndrome, sepsis, and COVID-19. Elevated short-term and long-term morbidity and mortality rates are well-known to be associated with these particular diseases. *Beta* phenotype, instead, was recognized by heterogeneous diseases: tachyarrhythmias, syncope, seizures, stroke, acute chest pain and poisoning. And lastly, *Gamma* phenotype showed syncope, acute chest pain, stroke, poisoning, orthopedic trauma, and seizures. The derived phenotypes typically assigns acute life-threatening diseases to the *Alpha* phenotype and distributing a priori less severe diseases or non-specific syndromic conditions to the remaining clusters. This classification results aligned with similar studies based on comparable methodologies, this is, one cluster pools critically ill cases.

Consistently, *Alpha* phenotype was associated to high-level on-scene advanced life support interventions, ICU-admissions, with rates of advanced airway management and intravenous medication. This contrast with *Beta* phenotype, and particularly with *Gamma* phenotype. Clinical evidence suggests that the association between non-unplanned mechanical ventilation and mortality are strong, just as concomitant administration of medication correlates with elevated major adverse events, thus supporting that the group of patients who meet these criteria are in the most critical category phenotype. As expected, the cluster with the poorest outcomes (*Alpha*) were elderly patients and more burdened by comorbidities. Several risk scores consider age and comorbidities as a vulnerability indicator, such as the aCCI. Frailty syndrome is a well-described multidimensional condition that, despite individual variability, constitutes a focal point directly related to poor outcomes. As age and comorbidities progress, physiological and psychosocial reserve may be jeopardized, enhancing clinical vulnerability.

One of the distinctive characteristics of this study was to conduct phenotyping with ultra-early bedside analytical data, based on prospective and standardized POCT. From the primitive capillary glucometers to the current POCT, technological advances have favored the production of novel devices, available on-scene, reduced dimensions, portable, ruggedized, and high-reliability, making an ideal solution for deployment in ambulances. Due to support provided by POCT, EMS-providers can obtain in a fast turnaround time crucial medical data, hitherto only retrieved at hospital level. Objective and structured clinical evaluation, combined with biomarker testing in acute life-threatening diseases, can help to guide targeted life support interventions on-scene or in route, and has optimized the decision-making process in prehospital critical care, aligned with international guidelines recommendations.

The main strength of the present invention is that phenotype *Alpha* comprises medically challenging situations, with some degree of frailty syndrome, and evident clinical disorders (impaired respiratory capacity, hemodynamic unsteadiness, neurological deterioration, lactic acidosis, hyperglycemia, etc.). This phenotype corresponds to 16% of cases, patients typically undergoing several advanced life support interventions on-scene, with a large proportion of inpatients, ICU-admission, and finally concluding with elevated mortality-related rates. This zero-minute flagging of high-risk patients, based not just on standard vital signs, but optimally supported by blood test biomarkers, empowers the EMS system to recognize patient potentially compromised and to proactively implement the necessary measures. In this sense, artificial intelligence represented a breakthrough, emerging phenotyping as a flexible and useful solution, with a proven risk-based case matching capability, allowing a massive data analysis in order to classify high-risk patients as a sentinel event.

In summary, unselected acute disease patients managed by EMS and transferred to ED can be categorized under three phenotypes, with different clinical and prognostic implications, based on data collected exclusively in prehospital care. By bedside phenotyping EMS-responders can know the risk-level, avoiding under-rated hidden unresolved health conditions and promoting well-characterization of complex or atypical clinical presentations. Identifying Alpha phenotype patients from the initial moments of assistance allows to develop a personalized strategy, tailoring the level of support and resources to individual situations, or even determining the most appropriate hospital for each patient. This knowledge provides valuable information for bedside decision-making from the outset, to design the best possible care strategy tailored to the individual case.

Particularly, the inventors of the present invention conducted a prospective, multicenter, EMS-delivered, ambulance-based, cohort study in adults with unselected acute disease, managed by EMS and evacuated with discharge-priority to emergency department, between January 1, 2020, and June 30, 2023, in Spain. The principal outcome was cumulative mortality (all-cause) at 2-, 7-, and 30-day. An unsupervised machine learning method (clustering) was used to determine the phenotypes. A total of 7,909 patients fulfilled the inclusion criteria. Three clusters were obtained: Alpha 16.2% (1281 patients), Beta 28.8% (2279), and Gamma 55% (4349). Alpha phenotype exhibited raised respiratory and cardiac rate, and decreased saturation, SaFi, blood pressure and Glasgow coma scale values. Beta phenotype and Gamma phenotype showed a gradual recovery to normal conditions. 2-day mortality was 18.6 %, 4.1 %, and 0.8 % respectively for Alpha, Beta and Gamma phenotype. And a 24.7 %, 6.2 %, and 1.7 % for 7-day mortality, and 33 %, 10.2 %, and 3.2 % for 30-day mortality.

It is important to highlight that the patients are on a prehospital care, in acute phase. In this context, there are significances changes in the variables measured on a prehospital care with respect to the variables measured in the hospital. Moreover, the reference values of some variables on a prehospital care are very different from the reference levels in a hospital (e.g., lactate).

Consequently, the present invention presents three differentiated phenotypes by using standard vital signs and blood test biomarkers at the prehospital scenario, the Alpha comprises high risk patients, followed by Beta and Gamma. This permits the EMS system a quick identification of patient potentially compromised and to proactively implement the necessary measures.

So, the first embodiment of the present invention refers to an evaluation system for establishing risk phenotypes regarding the clinical worsening of patients with acute time-dependent diseases and considering epidemiological variables, vital signs and/or blood test variables collected on a prehospital care, wherein the system comprises a processing unit configured to: a) Receiving data regarding epidemiological variables, vital signs and/or blood test variables collected on a prehospital care; b) process the data received regarding epidemiological variables, vital signs and/or blood test variables collected on a prehospital care for finding a statistically significant variations or deviations between the patients in acute phase that allow establishing risk phenotypes according to the clinical worsening of the patients in acute phase; and c) wherein the processing unit is characterized in that it is further configured to provide an output through a terminal classifying the patients in acute phase as pertaining to one out of three possible clusters according to their clinical worsening risk.

In a preferred embodiment, the worsening risk is mortality risk.

In a preferred embodiment, the system comprises a processing unit configured to: a) Receiving data regarding epidemiological variables, vital signs and/or blood test variables collected on a prehospital care; b) process the data received regarding epidemiological variables, vital signs and/or blood test variables collected on a prehospital care for finding a statistically significant variations or deviations between the patients in acute phase that allow establishing risk phenotypes according to the clinical mortality risk of the patients in acute phase; c) wherein the processing unit is characterized in that it is further configured to provide an output through a terminal classifying the patients in acute phase as pertaining to one out of three possible clusters according to their clinical mortality risk; and d) wherein the first cluster of patients in acute phase is characterized by having 2-days mortality risk of at least 4 %, 7-days mortality risk of at least 6 %, 30-days morality risk of at least 10%, or 365-days mortality risk of at least 30%; the second cluster of patients in acute phase is characterized by having 2-days mortality risk between 1 and 4 %, 7-days mortality risk between 5 and 7%, 30-days morality risk between 3 and 10%, or 365-days mortality risk between 3 and 20%; and the third cluster of patients in acute phase is characterized by having 2-days mortality risk of less than 1 %, 7-days mortality risk of less than 2%, 30-days morality risk of less than 3%, or 365-days mortality risk of less than 10%.

In a preferred embodiment, the system comprises a processing unit configured to: a) Receiving data regarding epidemiological variables, vital signs and/or blood test variables collected on a prehospital care; b) process the data received regarding epidemiological variables, vital signs and/or blood test variables collected on a prehospital care for finding a statistically significant variations or deviations between the patients in acute phase that allow establishing risk phenotypes according to the clinical mortality risk of the patients in acute phase; c) wherein the processing unit is characterized in that it is further configured to provide an output through a terminal classifying the patients in acute phase as pertaining to one out of three possible clusters according to their clinical mortality risk; and d) wherein the first cluster of patients in acute phase is characterized by having 2-days mortality risk of at least 18.6 %, 7-days mortality risk of at least 24.7 %, 30-days morality risk of at least 33%, or 365-days mortality risk of 50%; the second cluster of patients in acute phase is characterized by having 2-days mortality risk of at least 4.1 %, 7-days mortality risk of at least 6.2 %, 30-days morality risk of at least 10.2 %, or 365-days mortality risk of 20%; and the third cluster of patients in acute phase is characterized by having 2-days mortality risk of at least 0.8 %, 7-days mortality risk of at least 1.7 %, 30-days morality risk of at least 3.2 % or 365-days mortality risk of 5%.

The second embodiment of the present invention refers to a computer implemented method for establishing risk phenotypes regarding the clinical worsening of patients with acute time-dependent diseases considering epidemiological variables, vital signs and/or blood test variables collected on a prehospital care, which comprises: a) Receiving data regarding epidemiological variables, vital signs and/or blood test variables collected on a prehospital care; b) process the data received regarding epidemiological variables, vital signs and/or blood test variables collected on a prehospital care for finding a statistically significant variations or deviations between the patients in acute phase that allow establishing risk phenotypes according to the clinical worsening of the patients in acute phase; and c) wherein the processing unit is characterized in that it is further configured to provide an output through a terminal classifying the patients in acute phase as pertaining to one out of three possible clusters according to their clinical worsening risk.

In a preferred embodiment, the computer implemented method comprises: a) Receiving data regarding epidemiological variables, vital signs and/or blood test variables collected on a prehospital care; b) process the data received regarding epidemiological variables, vital signs and/or blood test variables collected on a prehospital care for finding a statistically significant variations or deviations between the patients in acute phase that allow establishing risk phenotypes according to the clinical mortality risk of the patients in acute phase; c) wherein the processing unit is characterized in that it is further configured to provide an output through a terminal classifying the patients in acute phase as pertaining to one out of three possible clusters according to their clinical mortality risk; and d) wherein the first cluster of patients in acute phase is characterized by having 2-days mortality risk of at least 4 %, 7-days mortality risk of at least 6 %, 30-days morality risk of at least 10%, or 365-days mortality risk of at least 30%; the second cluster of patients in acute phase is characterized by having 2-days mortality risk between 1 and 4 %, 7-days mortality risk between 5 and 7%, 30-days morality risk between 3 and 10%, or 365-days mortality risk between 3 and 20%; and the third cluster of patients in acute phase is characterized by having 2-days mortality risk of less than 1 %, 7-days mortality risk of less than 2%, 30-days morality risk of less than 3%, or 365-days mortality risk of less than 10%.

In a preferred embodiment, the computer implemented method comprises: a) Receiving data regarding epidemiological variables, vital signs and/or blood test variables collected on a prehospital care; b) process the data received regarding epidemiological variables, vital signs and/or blood test variables collected on a prehospital care for finding a statistically significant variations or deviations between the patients in acute phase that allow establishing risk phenotypes according to the clinical mortality risk of the patients in acute phase; c) wherein the processing unit is characterized in that it is further configured to provide an output through a terminal classifying the patients in acute phase as pertaining to one out of three possible clusters according to their clinical mortality risk; and d) wherein the first cluster of patients in acute phase is characterized by having 2-days mortality risk of at least 18.6 %, 7-days mortality risk of at least 24.7 %, 30-days morality risk of at least 33%, or 365-days mortality risk of 50%; the second cluster of patients in acute phase is characterized by having 2-days mortality risk of at least 4.1 %, 7-days mortality risk of at least 6.2 %, 30-days morality risk of at least 10.2 %, or 365-days mortality risk of 20%; and the third cluster of patients in acute phase is characterized by having 2-days mortality risk of at least 0.8 %, 7-days mortality risk of at least 1.7 %, 30-days morality risk of at least 3.2 % or 365-days mortality risk of 5%.

In a preferred embodiment, the processing unit is configured to select the most appropriate epidemiological variables, vital signs and/or blood test variables depending on which of the following types clinical worsening is predicted: mortality at 2, 7, 30, 365 days, hospital admission, ICU-admission, use of mechanical ventilation, or use of life-saving interventions.

In a preferred embodiment, the processing unit is characterized in that it is further configured to send or transmit the information obtained from the patient to the hospital in real time, once the patient has been classified as pertaining to one of the three clusters.

In a preferred embodiment, the epidemiological variable is the age, the vital sign is selected from: SaFi, mean blood pressure, facial expression and/or Glasgow coma scale, and the blood test variable is selected from: pH, lactate and/or creatinine.

In a preferred embodiment, the processing unit the epidemiological variable is selected from: Sex at birth, age, place of living, existence of Advanced Life Support and/nursing homes; the vital sign is selected from: Respiratory rate (breaths/min), Oxygen saturation (%), Fraction of inspired oxygen (%), SaFi, Systolic blood pressure (mmHg), Diastolic blood pressure (mmHg), Mean blood pressure (mmHg), Heart rate (beats/min), Temperature (°C), facial expression and/or Glasgow coma scale; and/or the blood test variables is selected from: pH, pCO2 (mmHg), pO2 (mmHg), Bicarbonate (mEq), Base excess (ecf) (mmol/L), TCO2 (mmol/L), Sodium (mmol/L), Potassium (mmol/L), Calcium (mmol/L), Chlorine (mmol/L), Hematocrit (%), Hemoglobin (g/dL), Glucose (mg/dL), Lactate (mmol/L), Creatinine (mg/dL), Blood urea nitrogen (mg/dL), Osmolarity (mOsm/Kg), GAP anion (mmol/L), Urinary anion (mmol/L), Potassium anion (mmol/L).

The third embodiment of the present invention refers to a computer implemented program comprising instructions which, when executed by a processing unit, configures the processing unit to execute the computer-implemented method of the invention.

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of". Thus, the phrase "consisting of" indicates that the listed elements are required or mandatory, and that no other elements may be present.

### Description of the figures

**Figure** 1. Study flow-chart.
Figure 2. Percentage of explained variances by the dimensions resulted from the principal component analysis used for the dimensionality reduction procedure.

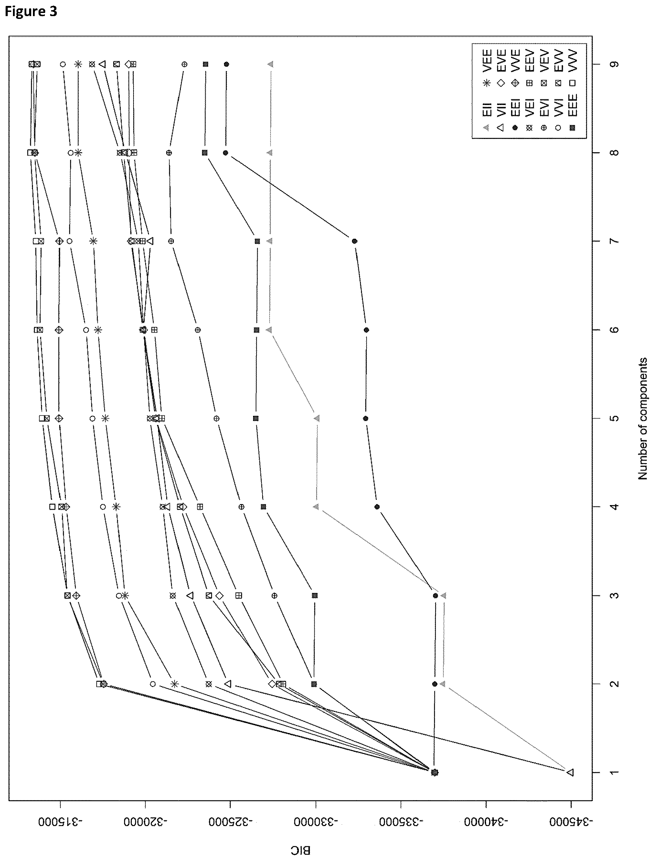

Figure 3. Bayesian Information Criterium for the different clustering number and methods. BIC: Bayesian Information criterion; EII: spherical, equal volume; VII: spherical, unequal volume; EEI: diagonal, equal volume and shape; VEI: diagonal, varying volume, equal shape; EVI: diagonal, equal volume, varying shape; VVI: diagonal, varying volume and shape; EEE: ellipsoidal, equal volume, shape, and orientation; VEE: ellipsoidal, equal shape and orientation; EVE: ellipsoidal, equal volume and orientation; VVE: ellipsoidal, equal orientation; EEV: ellipsoidal, equal volume and equal shape; VEV: ellipsoidal, equal shape; EVV: ellipsoidal, equal volume: VVV: ellipsoidal, varying volume, shape, and orientation.
Figure 4. Clustering-derived phenotypes. Green=Alpha, Red=Beta, Blue=Gamma phenotype.

### Detailed description of the invention

The present invention is illustrated by means of the Examples set below without the intention of limiting its scope of protection.

### Example 1. Material and methods

### Example 1.1. Study design and setting

A prospective, multicenter, EMS-delivered, ambulance-based, cohort study was conducted in adults with unselected acute disease, managed by EMS and evacuated with discharge-priority to ED, between January 1, 2020, and June 30, 2023.

The study involved the 1-1-2 emergency coordination center, six advance life support (ALS) units, thirty-eight basic life support (BLS) units, and four hospitals, in Salamanca, Segovia and Valladolid (Spain), comprising a population of 995,137 inhabitants, in cities and countryside. The Public Health System managed and coordinated all facilities. BLS are made up of two emergency medical technicians (EMT), and ALS, in addition, an emergency registered nurse (ERN), and a physician, operating all EMS-providers in compliance with basic advanced life support guidelines.

Cases were prospectively included uninterruptedly from two back-to-back studies conducted under identical and identified research protocols, "HITS study-" (ISRCTN48326533) and preBIO study" (ISRCTN49321933), approved by the institutional review board of the Public Health Service and followed the STrengthening the Reporting of OBservational studies in Epidemiology (STROBE) statement.

### Example 1.2. Population

Adults (>18 years) with unselected acute illness were screened for eligibility consecutively 24/7/365 by EMS. Additionally, following an evaluation by the ALS-physician, in order to be included in the study, patients had to be mandatorily referred to the Emergency Department (ED), either at BLS or at ALS.

Minors, pregnant women (evident or probable), cardiac arrest without recovery of spontaneous circulation on-scene, end-stage patients (documented by a report), impossibility to obtain prehospital blood tests (e.g., difficulty to establish venous access, breakdown of blood testing device), and no informed consent were excluded.

### Example 1.3. Outcome

The principal outcome was cumulative mortality (all-cause) at 2-, 7-, and 30-day. Secondary variables considered on-scene life support interventions (advanced airway management, defibrillation or pacemaker application, and intravenous medication delivery), suspected prehospital diagnoses (29 different sub-categories), hospital outcomes (inpatient, intensive care unit admission), and finally, the 17 comorbidities required to calculate the age-adjusted Charlson comorbidity index (aCCI), were collected.

### Example 1.4. Data collection, and processing

Covariates included epidemiological endpoints (sex at birth and age), on-scene vital signs (respiratory rate, oxygen saturation, blood pressure, heart rate, temperature, and Glasgow coma scale), and prehospital blood analysis (pH, bicarbonate, excess bases, sodium, potassium, chloride, calcium, hemoglobin, hematocrit, creatinine, blood urea nitrogen, glucose, and lactate), were picked up by ERN, based on the first encounter. Vital signs were obtained via LifePAK^{®} 15 monitor-defibrillator (Physio-Control, Inc., Redmond, USA), and blood tests by means of the epoc^{®} analyzer (Siemens Healthcare GmbH, Erlangen, Germany). Respiratory rate was monitored by direct observation and counting of breathing cycles for half a minute; in the case of very shallow or difficult breathing, respiratory rate was measured by direct auscultation. Administration of oxygen therapy (by any method) was also collected at the arrival of the ALS, so that once the fraction of inspired oxygen was known, the pulse oximetry saturation / fraction of inspired oxygen ratio (SaFi) was calculated.

After a 30-day follow-up period, data on mortality, comorbidities and hospital-admissions were collected by reviewing the electronic medical records. Data were recorded electronically in a database specifically designed for this purpose, recording the prehospital care variables. Access was by individual password and double authentication. Subsequent to data debugging (logic, range and consistency tests), a total of 54 variables were analyzed. Once the data were linked, the patient identifiers were anonymized.

### Example 1.5. Statistical analysis

Descriptive results and the associations between the outcomes and the analyzed variables were assessed by T-test, the Mann-Whitney U test or the chi-squared test, when appropriate. Absolute values and percentages were used for categorical variables were represented, and median interquartile ranges (IQR) were used for continuous variables because they did not follow a normal distribution. The clustering procedure was as follows: Firstly, a reduction of dimensionality (Principal Component Analysis) was used to reduce the number of variables. Then, the most parsimonious clustering model was selected by the Bayesian Information criterion (BIC) to perform a Gaussian Mixture Modelling for Model-Based Clustering. The number of clusters was fixed to three based on clinical criteria. Finally, as described at the beginning of this section each the resulting cluster were compared by including the outcomes, life support interventions, suspected prehospital diagnoses, and the aCCI.

Data was collected and registered in a database generated with the IBM SPSS Statistics for Apple version 20.0 software. (IBM Corp, Armonk USA). The caseload entry system was test-run to delete unclear or ambiguous items and to verify the adequacy of the data gathering system. Missing values were completely at random, therefore a listwise deletion method was used since it does not induce to biased means, variances or regression weights modification. The sample size needed for the clustering studies has been recently estimated [Dalmaijer, E.S., Nord, C.L, Astle, D.E. Statistical power for cluster analysis. BMC Bioinformatics 23, 205 (2022*)].* Due to the characteristics of the clustering procedure, this is, the phenotypes derived from clustering are driven by large effect sizes or by the accumulation of small effect sizes among the multiple variables analyzed, there is no effect of the covariance structure difference. Therefore, a small samples size (e.g., N=20), as stated in [Dalmaijer, E.S., Nord, C.L, Astle, D.E. Statistical power for cluster analysis. BMC Bioinformatics 23, 205 (2022*)*]*,* allows large cluster separations.

All calculations and analyses were performed by using our own codes, R packages [Scrucca L., Fop M., Murphy T. B. and Raftery A. E. (2016) mclust 5: clustering, classification and density estimation using Gaussian finite mixture models, The R Journal, 8/1, pp. 205-233*]* and base functions in R, version 4.2.2 (http://www.R-project.org; the R Foundation for Statistical Computing, Vienna, Austria).

### Example 2. Results

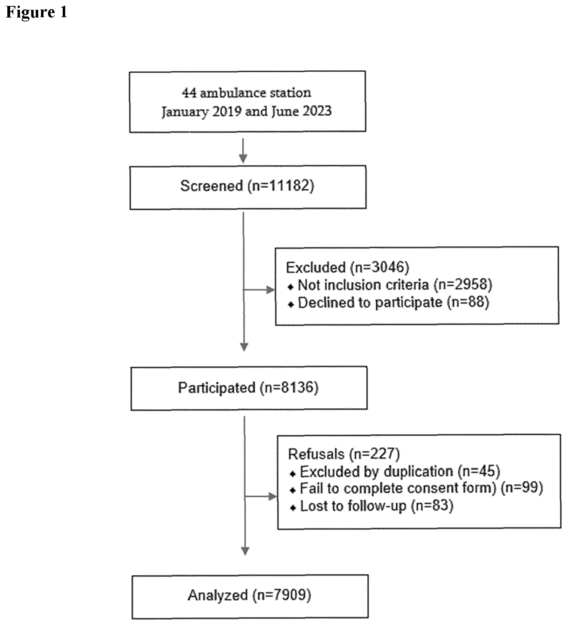

7,909 subjects satisfied inclusion criteria and were accepted for the final cohort analysis **(****Figure 1**). Clinical characterization based on unsupervised matching learning revealed three clinical phenotypes displaying marked contrasts. *Alpha* phenotype made up 16.2% (1281), with a median age of 74 years, 41.7% (534 cases) of females, an ALS evacuation rate of 76.3% (978 cases) and a nursing home origin of 21.7% (278 cases). *Beta* phenotype accounted 28.8 % (2279 cases), with a median age of 72 years and 39.8 % (906 cases) of females, 32.4 % of ALS evacuations and 11.8 % (269 cases) from nursing home origin. Lastly, *Gamma* phenotype was represented by 55 % (4349 cases), with a median age of 62 years, 42.3 % (1840 cases) of females, 41.5 % (1804 cases) of ALS transfers, and 6 % (1804 cases) from nursing homes (**Table 1**).

**Table 1. Clinical and biomarkers baseline patients' characteristics.**

| | | Phenotype | | | |
|---|---|---|---|---|---|
| | | Alpha | Beta | Gamma | p value^{b} |
| No. (%) with data^{a} | | 1281 (16.2) | 2279 (28.8) | 4349 (55) | N.A. |
| **Epidemiological variables** | | | | | |
| Sex at birth, female | | 534 (41.7) | 906 (39.8) | 1840 (42.3) | 0.132 |
| Age, year | | 74 (62-84) | 72 (56-82) | 62 (47-78) | <0.001 |
| Age groups, year | | | | | |
| | 18-49 | 148 (11.6) | 371 (16.3) | 1253 (28.8) | <0.001 |
| | 50-74 | 501 (39.1) | 887 (38.9) | 1740 (40) | |
| | >75 | 632 (49.3) | 1021 (44.8) | 1356 (31.2) | |
| Zone, rural | | 380 (29.7) | 564 (24.7) | 1109 (25.5) | 0.031 |
| Transfer, ALS | | 978 (76.3) | 739 (32.4) | 1804 (41.5) | <0.001 |
| Nursing homes | | 278 (21.7) | 269 (11.8) | 261 (6) | <0.001 |
| **On-scene vital signs** | | | | | |
| Respiratory rate, breaths/min | | 26 (17-33) | 18 (15-24) | 17 (14-19) | <0.001 |
| Oxygen saturation, % | | 86 (76-94) | 95 (92-97) | 98 (96-99) | <0.001 |
| Fraction of inspired oxygen, % | | 0.21 (0.21-0.31) | 0.21 (0.21-0.21) | 0.21 (0.21-0.21) | <0.001 |
| SaFi | | 343 (257-398) | 452 (438-462) | 467 (457-471) | <0.001 |
| Systolic blood pressure, mmHg | | 129 (99-155) | 133 (112-152) | 136 (119-153) | <0.001 |
| Diastolic blood pressure, mmHg | | 72 (55-89) | 77 (63-90) | 80 (69-91) | <0.001 |
| Mean blood pressure, mmHg | | 92 (71-110) | 96 (81-110) | 99 (87-111) | <0.001 |
| Heart rate, beats/min | | 103 (80-120) | 97 (77-121) | 79 (67-90) | <0.001 |
| Temperature, °C | | 36.2 (35.8-36.8) | 36.1 (35.9-36.7) | 36 (35.9-36.5) | <0.001 |
| Glasgow coma scale, points | | | | | |
| | Ocular | 4 (2-4) | 4 (4-4) | 4 (4-4) | <0.001 |
| | Verbal | 5 (2-5) | 5 (5-5) | 5 (5-5) | <0.001 |
| | Motor | 6 (4-6) | 6 (6-6) | 6 (6-6) | <0.001 |
| **Prehospital blood analysis** | | | | | |
| pH | | 7.31 (7.14-7.38) | 7.37 (7.32-7.42) | 7.39 (7.36-7.42) | <0.001 |
| pCO2, mmHg | | 48 (38-67) | 40 (34-46) | 39 (33-44) | <0.001 |
| pO2, mmHg | | 23 (17-35) | 30 (22-41) | 32 (23-41) | <0.001 |
| Bicarbonate, mEq | | 22 (18-27) | 23 (20-26) | 24 (22-27) | <0.001 |
| Base excess (ecf), mmol/L | | -1.8 (-7.2; 2.4) | -0.3 (-3; 1.7) | 0.7 (-1.6; 1.9) | <0.001 |
| TCO2, mmol/L | | 27 (22-34) | 26 (22-29) | 26 (23-28) | <0.001 |
| Sodium, mmol/L | | 139 (136-141) | 139 (136-141) | 139 (137-141) | <0.001 |
| Potassium, mmol/L | | 4.2 (3.9-5) | 4.1 (3.8-4.6) | 4.1 (3.8-4.4) | <0.001 |
| Calcium, mmol/L | | 1.14 (1.04-1.22) | 1.14 (1.08-1.21) | 1.14 (1.08-1.21) | 0.039 |
| Chlorine, mmol/L | | 103 (100-107) | 103 (100-106) | 103 (100-105) | 0.011 |
| Hematocrit, % | | 41 (36-45) | 41 (38-45) | 42 (39-45) | <0.001 |
| Hemoglobin, g/dL | | 13.8 (12.1-15.7) | 14 (12.6-15.7) | 14.2 (13-15.7) | <0.001 |
| Glucose, mg/dL | | 183 (130-275) | 160 (120-197) | 113 (99-132) | <0.001 |
| Lactate, mmol/L | | 3.29 (2.13-6.29) | 2.43 (1.59-3.74) | 1.83 (1.13-2.82) | <0.001 |
| Creatinine, mg/dL | | 1.21 (0.88-1.86) | 0.96 (0.79-1.28) | 0.86 (0.75-1.08) | <0.001 |
| Blood urea nitrogen, mg/dL | | 24 (16-37) | 18 (13-26) | 14 (11-20) | <0.001 |
| Osmolarity, mOsm/Kg | | 298 (290-307) | 294 (288-299) | 290 (286-294) | <0.001 |
| GAP anion, mmol/L | | 11.8 (7.5-16.6) | 11.8 (8.3-15.6) | 11.5 (8.4-14.6) | 0.026 |
| Urinary anion, mmol/L | | 39.9 (36.1-43.2) | 40 (36.8-42.8) | 40.3 (37.5-42.8) | 0.002 |
| Potassium anion, mmol/L | | 16.2 (11.9-21.3) | 16 (12.6-17.9) | 15.7 (12.6-18.7) | <0.001 |

| | | | | | |
|---|---|---|---|---|---|
| ***Abbreviations: NA: not applicable; ALS: advanced life support; SaFi: ratio: pulse oximetry saturation* / *fraction of inspired oxygen ratio; pCO2: partial pressure of carbon dioxide; pO2: partial pressure of oxygen; TCO2: total carbon dioxide content.aValues expressed as total number (percentage) and medians (25 percentile-75 percentile), as appropriate.bThe Mann-Whitney U test or Chi-squared test was used as appropriate.*** | | | | | |

On-scene vital signs also evidenced significant differences between clusters. *Alpha* phenotype exhibited raised respiratory and cardiac rate, and decreased saturation, SaFi, blood pressure and Glasgow coma scale values (p<0.001 in. all cases). In contrast, *Beta* phenotype, and especially *Gamma* phenotype, showed a gradual recovery to normal conditions (**Table 1**). Differences across phenotypes were also evident in blood biomarkers, with significant differences among other parameters in pH, partial pressure of carbon dioxide, lactate, creatinine, and glucose (p<0.001 in all) (**Table 1**).

The uncovering of outcomes across clusters was as follows: 2-day mortality was 18.6 %, 4.1 %, and 0.8 % respectively for *Alpha, Beta* and *Gamma* phenotype. And a 24.7 %, 6.2 %, and 1.7 % for 7-day mortality, and 33 %, 10.2 %, and 3.2 % for 30-day mortality (**Table 2**).

**Table 2. Principal outcomes, and other determinants.**

| | Phenotype | | | | |
|---|---|---|---|---|---|
| | Alpha | Beta | Gamma | p value^{b} | |
| No. (%) with data^{a} | 1281 (16.2) | 2279 (28.8) | 4349 (55) | N.A. | |
| **Support on-scene** | | | | | |
| NIMV | 246 (19.2) | 41 (1.8) | 6 (0.1) | <0.001 | |
| IMV | 260 (20.3) | 130 (5.7) | 95 (2.2) | <0.001 | |
| Pacemaker | 26 (2) | 34 (1.5) | 42 (1) | 0.007 | |
| Cardioversion | 19 (1.5) | 60 (2.6) | 3 (0.1) | <0.001 | |
| Defibrillation | 75 (5.9) | 12 (0.5) | 8 (0.2) | | |
| Intravenous medication, quantity | | | | | |
| | No medication | 52 (4.1) | 416 (18.3) | 1316 (30.3) | <0.001 |
| | 1 | 122 (9.5) | 587 (25.8) | 1258 (28.9) | |
| | 2 | 176(13.7) | 427 (18.7) | 837 (19.2) | |
| | 3 | 204 (15.9) | 363 (15.9) | 467 (10.7) | |
| | 4 | 219 (17.1) | 243 (10.7) | 272 (6.3) | |
| | 5 | 222 (17.3) | 133 (5.8) | 125 (2.9) | |
| | 6 | 163 (12.7) | 66 (2.9) | 57 (1.3) | |
| | 7 or more | 123 (9.6) | 44 (1.9) | 17 (0.4) | |
| Vasoactive agents | | 145 (11.3) | 36 (1.6) | 11 (0.3) | <0.001 |
| **Suspected prehospital diagnoses** | | | | | |
| Abdominal pain/GB | | 22 (1.7) | 107 (4.7) | 213 (4.9) | <0.001 |
| Abdominal trauma | | 2 (0.2) | 13 (0.6) | 16 (0.4) | |
| Acute chest pain | | 19 (1.5) | 158 (6.9) | 578 (13.3) | |
| Acute myocardial infarction | | 52 (4.1) | 134 (5.9) | 278 (6.4) | |
| Anaphylaxis | | 16 (1.2) | 24 (1.1) | 41 (0.9) | |
| Bradyarrhythmia | | 9 (0.7) | 31 (1.4) | 44(1) | |
| Burns | | 6 (0.5) | 9 (0.4) | 19 (0.4) | |
| Cardiac arrest | | 117 (9.1) | 14 (0.6) | 7 (0.2) | |
| Confusional syndrome | | 3 (0.2) | 18 (0.8) | 28 (0.6) | |
| Congestive heart failure | | 104 (8.1) | 15 (0.7) | 2(0) | |
| COPD/dyspnea | | 237 (18.5) | 125 (5.5) | 86(2) | |
| Headache | | 2 (0.2) | 1 (0) | 32 (0.7) | |
| Heart failure | | 137 (10.7) | 102 (4.5) | 46 (1.1) | |
| Hypertensive crisis | | 10 (0.8) | 18 (0.8) | 87 (2) | |
| Infection/febrile syndrome | | 74 (5.8) | 120 (5.3) | 125 (2.9) | |
| Metabolic disease | | 64 (5) | 56 (2.5) | 22 (0.5) | |
| Orthopedic trauma | | 3 (0.2) | 53 (2.3) | 346 (8) | |
| Poisoning | | 41 (3.2) | 155 (6.8) | 419 (9.6) | |
| Poly traumatized | | 44 (3.4) | 58 (2.5) | 60 (1.4) | |
| SARS-CoV-2 | | 50 (3.9) | 49 (2.2) | 60 (1.4) | |
| Seizures | | 35 (2.7) | 182 (8) | 329 (7.6) | |
| Sepsis | | 82 (6.4) | 56 (2.5) | 17 (0.4) | |
| Status epilepticus | | 8 (0.6) | 15 (0.7) | 9 (0.2) | |
| Stroke | | 49 (3.8) | 175 (7.7) | 420 (9.7) | |
| Syncope | | 35 (2.7) | 224 (9.8) | 589 (13.5) | |
| Tachyarrhythmia | | 23 (1.8) | 234 (10.3) | 50 (1.1) | |
| Thoracic trauma | | 7 (0.5) | 21 (0.9) | 55 (1.3) | |
| Transient ischemic attack | | 3 (0.2) | 32 (1.4) | 137 (3.2) | |
| Trauma brain injury | | 31 (2.4) | 79 (3.5) | 234 (5.4) | |
| **Hospital outcomes** | | | | | |
| aCCI, points | | 6 (4-9) | 5 (3-7) | 3 (1-5) | <0.001 |
| Inpatient | | 1108 (86.5) | 1345 (59) | 1793 (41.2) | <0.001 |
| ICU-admission | | 339 (26.5) | 281 (12.3) | 273 (6.3) | <0.001 |
| ACCU-admission | | 124 (9.7) | 200 (8.8) | 325 (7.5) | 0.021 |
| Stroke unit-admission | | 15 (1.2) | 110 (4.8) | 289 (6.6) | <0.001 |
| Mortality | | | | | |
| | 2-day | 238 (18.6) | 93 (4.1) | 33 (0.8) | <0.001 |
| | 7-day | 316 (24.7) | 142 (6.2) | 74 (1.7) | <0.001 |
| | 30-day | 423 (33) | 233 (10.2) | 137 (3.2) | <0.001 |

| | | | | | |
|---|---|---|---|---|---|
| Abbreviations: NA: not applicable; NIMV: non-invasive mechanical ventilation; IMV: invasive mechanical ventilation; GB: gastrointestinal bleeding; COPD: chronic obstructive pulmonary disease; SARS-CoV-2: severe acute respiratory syndrome coronavirus 2; aCCI: Age adjusted Charlson comorbidity index; ICU: intensive care unit; ACCU: Acute Cardiac Care Unit. ^{a}Values expressed as total number (percentage) and medians (25 percentile-75 percentile), as appropriate. ^{b}The Mann-Whitney U test or Chi-squared test was used as appropriate. | | | | | |

In addition to mortality disparities, *Alpha* phenotype stood out due to an increased requirement for on-scene advanced life support interventions, associated burden of comorbidities, and major ICU-admissions.

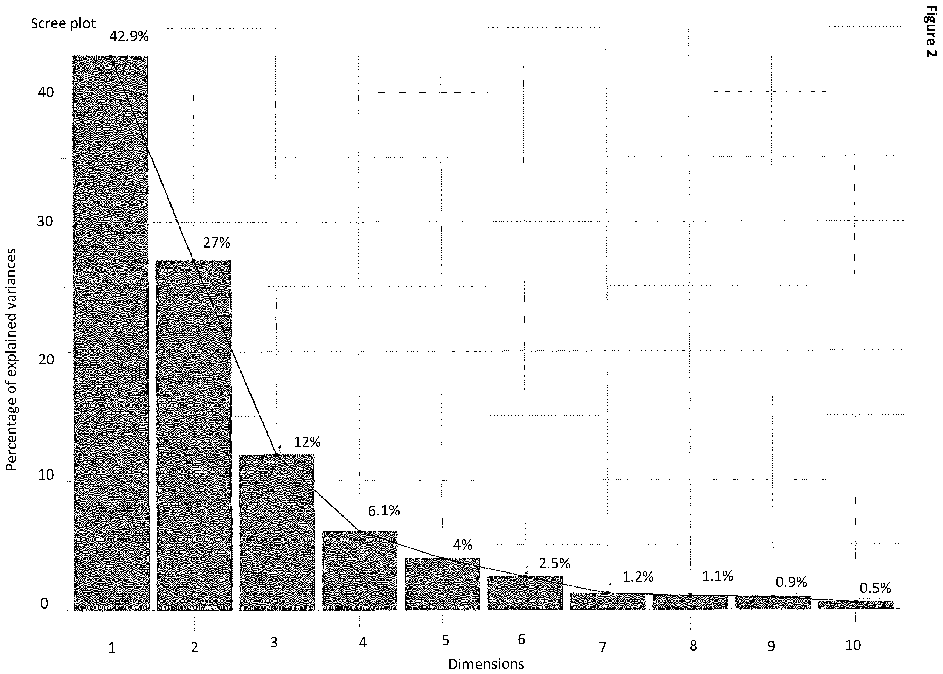

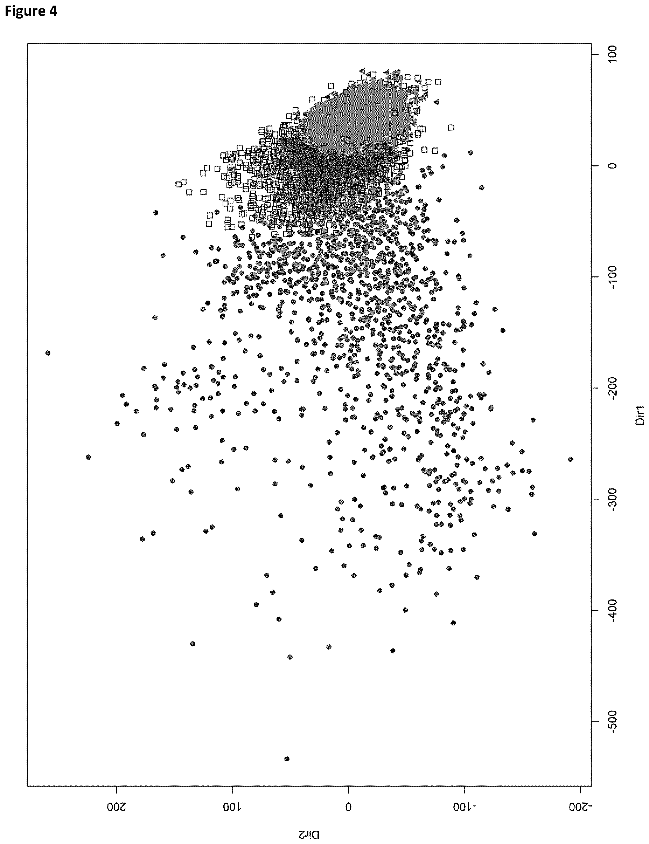

The clustering procedure was preceded by a reduction of dimensionality. As can be observed in **Figure 2** the first three dimensions explained the 81,9 % of variance. The principal component analysis output was then used for the clustering procedure, as shown by **Figure 3** the most parsimonious clustering model was the ellipsoidal, varying volume, shape, and orientation (VVV). Moreover, when increasing the number of clusters, the clustering model was stable and no major difference on BIC was found, suggesting that VVV model and the clinically selected number of clusters was supported by BIC results. The cluster results are shown in **Figure 4****.**

## Claims

1. An evaluation system for establishing risk phenotypes regarding the clinical worsening of patients with acute time-dependent diseases and considering epidemiological variables, vital signs and/or blood test variables collected on a prehospital care, wherein the system comprises a processing unit configured to:
a) Receiving data regarding epidemiological variables, vital signs and/or blood test variables collected on a prehospital care;
b) Process the data received regarding epidemiological variables, vital signs and/or blood test variables collected on a prehospital care for finding a statistically significant variations or deviations between the patients in acute phase that allow establishing risk phenotypes according to the clinical worsening of the patients in acute phase;
c) Wherein the processing unit is **characterized in that** it is further configured to provide an output through a terminal classifying the patients in acute phase as pertaining to one out of three possible clusters according to their clinical worsening risk.

2. Evaluation system, according to claim 1, wherein the system comprises a processing unit configured to:
a) Receiving data regarding epidemiological variables, vital signs and/or blood test variables collected on a prehospital care;
b) Process the data received regarding epidemiological variables, vital signs and/or blood test variables collected on a prehospital care for finding a statistically significant variations or deviations between the patients in acute phase that allow establishing risk phenotypes according to the clinical mortality risk of the patients in acute phase;
c) Wherein the processing unit is **characterized in that** it is further configured to provide an output through a terminal classifying the patients in acute phase as pertaining to one out of three possible clusters according to their clinical mortality risk; and
d) Wherein the first cluster of patients in acute phase is **characterized by** having 2-days mortality risk of at least 4 %, 7-days mortality risk of at least 6 %, 30-days morality risk of at least 10%, or 365-days mortality risk of at least 30%; the second cluster of patients in acute phase is **characterized by** having 2-days mortality risk between 1 and 4 %, 7-days mortality risk between 5 and 7%, 30-days morality risk between 3 and 10%, or 365-days mortality risk between 3 and 20%; and the third cluster of patients in acute phase is **characterized by** having 2-days mortality risk of less than 1 %, 7-days mortality risk of less than 2%, 30-days morality risk of less than 3%, or 365-days mortality risk of less than 10%.

3. Evaluation system, according any of the previous claims, wherein the system comprises a processing unit configured to:
a) Receiving data regarding epidemiological variables, vital signs and/or blood test variables collected on a prehospital care;
b) Process the data received regarding epidemiological variables, vital signs and/or blood test variables collected on a prehospital care for finding a statistically significant variations or deviations between the patients in acute phase that allow establishing risk phenotypes according to the clinical mortality risk of the patients in acute phase;
c) Wherein the processing unit is **characterized in that** it is further configured to provide an output through a terminal classifying the patients in acute phase as pertaining to one out of three possible clusters according to their clinical mortality risk; and
d) Wherein the first cluster of patients in acute phase is **characterized by** having 2-days mortality risk of at least 18.6 %, 7-days mortality risk of at least 24.7 %, 30-days morality risk of at least 33%, or 365-days mortality risk of 50%; the second cluster of patients in acute phase is **characterized by** having 2-days mortality risk of at least 4.1 %, 7-days mortality risk of at least 6.2 %, 30-days morality risk of at least 10.2 %, or 365-days mortality risk of 20%; and the third cluster of patients in acute phase is **characterized by** having 2-days mortality risk of at least 0.8 %, 7-days mortality risk of at least 1.7 %, 30-days morality risk of at least 3.2 % or 365-days mortality risk of 5%.

4. Evaluation system, according to any of the previous claims, wherein the processing unit configured to select the most appropriate epidemiological variables, vital signs and/or blood test variables depending on which of the following types clinical worsening is predicted: mortality at 2, 7, 30, 365 days, hospital admission, ICU-admission, use of mechanical ventilation, or use of life-saving interventions.

5. Evaluation system, according to any of the previous claims, wherein the processing unit is **characterized in that** it is further configured to send or transmit the information obtained from the patient to the hospital in real time, once the patient has been classified as pertaining to one of the three clusters.

6. Evaluation system, according to any of the previous claims, wherein the epidemiological variable is the age, the vital sign is selected from: SaFi, mean blood pressure, facial expression and/or Glasgow coma scale, and the blood test variable is selected from: pH, lactate and/or creatinine.

7. Evaluation system, according to any of the previous claims, wherein the epidemiological variable is selected from: Sex at birth, age, place of living, existence of Advanced Life Support and/nursing homes; the vital sign is selected from: Respiratory rate (breaths/min), Oxygen saturation (%), Fraction of inspired oxygen (%), SaFi, Systolic blood pressure (mmHg), Diastolic blood pressure (mmHg), Mean blood pressure (mmHg), Heart rate (beats/min), Temperature (°C), facial expression and/or Glasgow coma scale; and/or the blood test variables is selected from: pH, pCO2 (mmHg), pO2 (mmHg), Bicarbonate (mEq), Base excess (ecf) (mmol/L), TCO2 (mmol/L), Sodium (mmol/L), Potassium (mmol/L), Calcium (mmol/L), Chlorine (mmol/L), Hematocrit (%), Hemoglobin (g/dL), Glucose (mg/dL), Lactate (mmol/L), Creatinine (mg/dL), Blood urea nitrogen (mg/dL), Osmolarity (mOsm/Kg), GAP anion (mmol/L), Urinary anion (mmol/L), Potassium anion (mmol/L).

8. A computer implemented method for establishing risk phenotypes regarding the clinical worsening of patients with acute time-dependent diseases considering epidemiological variables, vital signs and/or blood test variables collected on a prehospital care, which comprises:
a) Receiving data regarding epidemiological variables, vital signs and/or blood test variables collected on a prehospital care;
b) Process the data received regarding epidemiological variables, vital signs and/or blood test variables collected on a prehospital care for finding a statistically significant variations or deviations between the patients in acute phase that allow establishing risk phenotypes according to the clinical worsening of the patients in acute phase;
c) Wherein the processing unit is **characterized in that** it is further configured to provide an output through a terminal classifying the patients in acute phase as pertaining to one out of three possible clusters according to their clinical worsening risk.

9. Computer implemented method, according to claim 8, which comprises:
a) Receiving data regarding epidemiological variables, vital signs and/or blood test variables collected on a prehospital care;
b) Process the data received regarding epidemiological variables, vital signs and/or blood test variables collected on a prehospital care for finding a statistically significant variations or deviations between the patients in acute phase that allow establishing risk phenotypes according to the clinical mortality risk of the patients in acute phase;
c) Wherein the processing unit is **characterized in that** it is further configured to provide an output through a terminal classifying the patients in acute phase as pertaining to one out of three possible clusters according to their clinical mortality risk; and
d) Wherein the first cluster of patients in acute phase is **characterized by** having 2-days mortality risk of at least 4 %, 7-days mortality risk of at least 6 %, 30-days morality risk of at least 10%, or 365-days mortality risk of at least 30%; the second cluster of patients in acute phase is **characterized by** having 2-days mortality risk between 1 and 4 %, 7-days mortality risk between 5 and 7%, 30-days morality risk between 3 and 10%, or 365-days mortality risk between 3 and 20%; and the third cluster of patients in acute phase is **characterized by** having 2-days mortality risk of less than 1 %, 7-days mortality risk of less than 2%, 30-days morality risk of less than 3%, or 365-days mortality risk of less than 10%.

10. Computer implemented method, according any of the claims 8 or 9, which comprises:
a) Receiving data regarding epidemiological variables, vital signs and/or blood test variables collected on a prehospital care;
b) Process the data received regarding epidemiological variables, vital signs and/or blood test variables collected on a prehospital care for finding a statistically significant variations or deviations between the patients in acute phase that allow establishing risk phenotypes according to the clinical mortality risk of the patients in acute phase;
c) Wherein the processing unit is **characterized in that** it is further configured to provide an output through a terminal classifying the patients in acute phase as pertaining to one out of three possible clusters according to their clinical mortality risk; and
d) Wherein the first cluster of patients in acute phase is **characterized by** having 2-days mortality risk of at least 18.6 %, 7-days mortality risk of at least 24.7 %, 30-days morality risk of at least 33%, or 365-days mortality risk of 50%; the second cluster of patients in acute phase is **characterized by** having 2-days mortality risk of at least 4.1 %, 7-days mortality risk of at least 6.2 %, 30-days morality risk of at least 10.2 %, or 365-days mortality risk of 20%; and the third cluster of patients in acute phase is **characterized by** having 2-days mortality risk of at least 0.8 %, 7-days mortality risk of at least 1.7 %, 30-days morality risk of at least 3.2 % or 365-days mortality risk of 5%.

11. Computer implemented method, according to any of the claims 8 to 10, wherein the processing unit configured to select the most appropriate epidemiological variables, vital signs and/or blood test variables depending on which of the following types clinical worsening is predicted: mortality at 2, 7, 30, 365 days, hospital admission, ICU-admission, use of mechanical ventilation, or use of life-saving interventions.

12. Computer implemented method, according to any of the claims 8 to 11, wherein the processing unit is **characterized in that** it is further configured to send or transmit the information obtained from the patient to the hospital in real time, once the patient has been classified as pertaining to one of the three clusters.

13. Computer implemented method, according to any of the claims 8 to 12, wherein the epidemiological variable is the age, the vital sign is selected from: SaFi, mean blood pressure, facial expression and/or Glasgow coma scale, and the blood test variable is selected from: pH, lactate and/or creatinine.

14. Computer implemented method, according to any of the claims 8 to 13, wherein the epidemiological variable is selected from: Sex at birth, age, place of living, existence of Advanced Life Support and/nursing homes; the vital sign is selected from: Respiratory rate (breaths/min), Oxygen saturation (%), Fraction of inspired oxygen (%), SaFi, Systolic blood pressure (mmHg), Diastolic blood pressure (mmHg), Mean blood pressure (mmHg), Heart rate (beats/min), Temperature (°C), facial expression and/or Glasgow coma scale; and/or the blood test variables is selected from: pH, pCO2 (mmHg), pO2 (mmHg), Bicarbonate (mEq), Base excess (ecf) (mmol/L), TCO2 (mmol/L), Sodium (mmol/L), Potassium (mmol/L), Calcium (mmol/L), Chlorine (mmol/L), Hematocrit (%), Hemoglobin (g/dL), Glucose (mg/dL), Lactate (mmol/L), Creatinine (mg/dL), Blood urea nitrogen (mg/dL), Osmolarity (mOsm/Kg), GAP anion (mmol/L), Urinary anion (mmol/L), and/or Potassium anion (mmol/L).

15. A computer implemented program comprising instructions which, when executed by a processing unit, configures the processing unit to execute the computer-implemented method according to any one of claims 8 to 14.
